# EUROPÄISCHE PATENTSCHRIFT

(11) **EP 0 634 015 B1**
(45) Veröffentlichungstag und Bekanntmachung des Hinweises auf die Patenterteilung: **10.09.1997**
(21) Anmeldenummer: 93905315.3
(22) Anmeldetag: 08.03.1993
(51) Int. Cl.: G01N 33/543, G01N 33/538, B01L 3/00

(54) **EINWEGREAKTIONSGEFÄSS FÜR DIE FESTPHASENIMMUNANALYTIK UND VERFAHREN ZUR MESSUNG VON ÜBER IMMUNREAKTIONEN BESTIMMBAREN KOMPONENTEN**
ONE-WAY REACTION VESSEL FOR THE SOLID-PHASE IMMUNOLOGICAL ANALYSIS OF, AND A METHOD OF MEASURING CONSTITUENTS WHICH CAN BE DETERMINED VIA IMMUNE REACTIONS
REACTEUR A USAGE UNIQUE POUR L'ANALYSE IMMUNOLOGIQUE EN PHASE SOLIDE ET PROCEDE VISANT A MESURER LES COMPOSANTS DOSABLES PAR IMMUNOREACTIONS

(30) Priorität: 18.03.1992 DE 4208732
(43) Veröffentlichungstag der Anmeldung: 18.01.1995
(73) Patentinhaber: ABION Beteiligungs- und Verwaltungsgesellschaft mbH, 52428 Jülich (DE)
(72) Erfinder: ERHARDT, Ursula, D-8139 Bernried (DE)
(74) Vertreter: Meyers, Hans-Wilhelm, Dr.Dipl.-Chem.
(86) Internationale Anmeldenummer: EP9300520
(87) Internationale Veröffentlichungsnummer: WO9319368

(56) Entgegenhaltungen:
- WO-A-86/03589
- WO-A-88/09201
- WO-A-91/13354
- WO-A-92/05442
- DE-A- 3 733 098
- US-A- 4 039 652
- US-A- 4 145 406
- US-A- 4 892 710
- CHEMICAL ABSTRACTS, Band 110, Nr. 23, 05 Juni 1989, Columbus, OH (US); Seite 382, Nr. 208819/

## Beschreibung

Die vorliegende Erfindung betrifft ein Einwegreaktionsgefäß für die Festphasenimmunanalytik sowie ein Verfahren, bei dem das Einwegreaktionsgefäß verwendet wird.

Affinitätschromatographie ist eine Technik, die bei der präparativen Reinigung von Biomolekülen breite Anwendung findet. Dabei wird eine spezifische Wechselwirkung des zu bestimmenden Moleküls mit einem dazu komplementären Bindungspartner ausgenutzt. Zur praktischen Durchführung solcher Methoden wird in der Regel eine Probe, die das zu reinigende Biomolekül enthält, auf eine Chromatographiesäule gegeben, die auf einem festen Trägermaterial einen der zueinander komplementären Bindungspartner gebunden enthält. Als Paare komplementärer Bindungspartner sind beispielsweise Enzyme und deren Substrate, Antikörper und Hapten bzw. Antigen, sowie zueinander komplementäre DNA- oder RNA- Einzelstränge zu nennen.

Bei der Immunaffinitätschromatographie wird die Wechselwirkung zwischen einem Antigen bzw. Hapten und dem dazugehörigen Antikörper zur Bestimmung ausgenutzt.

Bei Immuno-Assays unter Verwendung der Affinitätschromatographie wurden bisher im wesentlichen wiederverwendbare Säulen, die mit dem Antikörper oder Antigen (im folgenden als Liganden bezeichnet), gebunden an einen festen Träger, gepackt sind, verwendet. Um die zügige Durchführung des Chromatographievorgangs zu gewährleisten, wird die zu analysierende Probe unter hohem Druck durch die Chromatographiesäule gepreßt. Die Anwendung der Hochdruckflüssigkeitschromatographie (HPLC) im Rahmen von Immuno-Assays wird beispielsweise beschrieben von de Elvis, W.U. und Wilson, G.S. in "Analytical Chemistry", 1985, Vol. 57, Seiten 2754-2756, wie auch von Sportsman, J.R. et al. in "Analytical Chemistry", 1983, Vol. 55, 771-775. Der Einsatz der HPLC zur Durchführung von Immuno-Assays hat jedoch mehrere Nachteile. So macht diese Technik einerseits den Einsatz aufwendiger Apparaturen notwendig, und andererseits werden an die zur Durchführung der Analyse benötigten Chromatographiesäulen hohe technische Anforderungen gestellt. Die Säulen müssen sowohl den bei der HPLC auftretenden hohen Drücken standhalten, als auch wiederverwendbar sein, um ein rationelles Arbeiten bei dieser Technik zu ermöglichen. Die Wiederverwendung der Säule erfordert jedoch zusätzliche Maßnahmen, bevor eine erneute Probe auf derselben Säule analysiert werden kann. Wie in der DE-OS 37 11 894 offenbart, werden wiederverwendbare Säulen vor jeder Analyse regeneriert, um den Einfluß mitgeschleppter und nicht ausreichend eluierter Substanzen aus dem vorhergehenden Versuch auszuschalten.

Aus der DE-OS 24 48 411 ist ein Reaktionsgefäß für die Festphasenimmunanalytik bekannt. Das für mehrfache Verwendung ausgelegte Gefäß macht jedoch vor einer quantitativen Bestimmung eine Eichung mit Standards der zu bestimmenden Komponente erforderlich. Weiterhin sind bei dem in dieser Schrift offenbarten Reaktionsgefäß lange Inkubationszeiten notwendig, während denen der Durchfluß durch die Säule gestoppt ist, empfohlenerweise 6 Stunden bis 2 Tage, um eine ausreichende Bindung der zu bestimmenden Komponente an die auf den Träger aufgebrachte immunologische Reaktivkomponente sicherzustellen.

Die Bindungsreaktion wird jedoch immer vor Erreichen des Bindungsgleichgewichts, das 1 - 2 Tage erfordert, abgebrochen, um ein rationelles Arbeiten zu gewährleisten. Auch dieses frühzeitige Abbrechen der Bindungsreaktion hat zur Folge, daß zur Eichung eine Vergleichsanalytik mit Standardlösungen durchgeführt werden muß.

Entsprechend der Bevorzugung von Wegwerfsäulen in der Praxis beschreibt die DE-PS 26 26 823 ein Verfahren zur Herstellung einer vorgefertigten Wegwerf-Adsorptionssäule insbesondere für Radioimmunoassays unter Verwendung von Analysiergeräten, die mit Zentrifugalkräften arbeiten. Diese Säule ist aber keine Reaktionssäule und nur zur Abtrennung nach einer Immunreaktion geeignet, da sie nicht mit einer spezifischen Immunreaktivkomponente beladen wird. Um Störeffekte bei der Trennung mittels Gelbett zu vermeiden, wird der Strömungswiderstand der porösen Rückhalteglieder möglichst niedrig gehalten.

Die WO 92/05442 ist ein Dokument des Standes der Technik gemäß Art. 54(3) EPÜ und schlägt ein Einwegreaktionsgefäß für die Festphasenimmunanalytik vor, welches eine einfache Handhabung erlaubt und universell anwendbar ist im Rahmen von bekannten Verfahren zur quantitativen Bestimmung von immunologisch reaktiven Komponenten. Das Einwegreaktionsgefäß ist zum sofortigen Gebrauch ohne vorherige Kalibrierung oder Regenerierung des darin befindlichen Trägermaterials mit der immunologischen Reaktivkomponente geeignet. Das Einwegreaktionsgefäß eignet sich zur Durchführung manueller wie auch voll automatisierter Probenanalysen. Das Trägermaterial ist dabei zwischen zwei Einrichtungen, die den Durchfluß kontrollieren, in einem im wesentlichen zylindrischen Hohlkörper angeordnet.

Aufgabe der vorliegenden Erfindung ist es, ein Einwegreaktionsgefäß für die Festphasenimmunanalytik zur Verfügung zu stellen, das die rasche direkte Durchführung von Immuno-Assays ermöglicht, das einfach gehandhabt werden kann, und das zum sofortigen Gebrauch, ohne vorherige Kalibrier- und Regeneriermaßnahmen, geeignet ist, sowie ein Verfahren zur Verwendung dieses Gefäßes.

Diese Aufgabe wird vorrichtungsgemäß gelöst durch ein oben und unten offenes Einwegreaktionsgefäß für die Festphasenimmunanalytik mit mindestens einer auf ein Trägermaterial aufgebrachten immunologischen Reaktivkomponente, wobei das Trägerbettvolumen bis zu 600 µl beträgt, und wobei die Durchflußgeschwindigkeit der Flüssigkeiten ausschließlich vom Trägermaterial bestimmt wird. Das Trägermaterial liegt entweder selbst in Form einer festen Fritte, Membran o.ä. vor, oder es wird von einer unteren und oberen porösen Trenneinrichtung begrenzt.

Verfahrensgemäß wird diese Aufgabe gelöst durch ein Verfahren zur Bestimmung von über eine Immunreaktion bestimmbaren Komponenten, bei dem auf ein erfindungsgemäßes Einwegreaktionsgefäß eine zu analysierende Probe gegeben wird, die zu bestimmende Komponente in der Probe von der komplementären immunologischen Reaktivkomponete in dem Einwegreaktionsgefäß während des direkten Durchflusses zurückgehalten wird, und die zu bestimmende Komponente
a) eluiert und bestimmt wird oder
b) im Einwegreaktionsgefäß über Verstärkerreaktionen mit immunologischen Markern markiert und dann bestimmt wird oder
c) über eine zu der zu bestimmenden Komponente konkurrierende markierte Verbindung bestimmt wird.

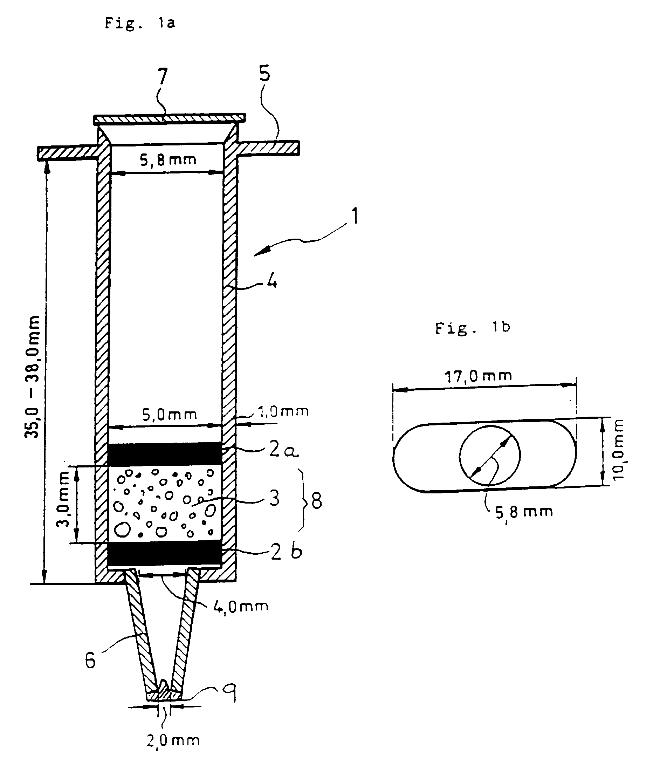

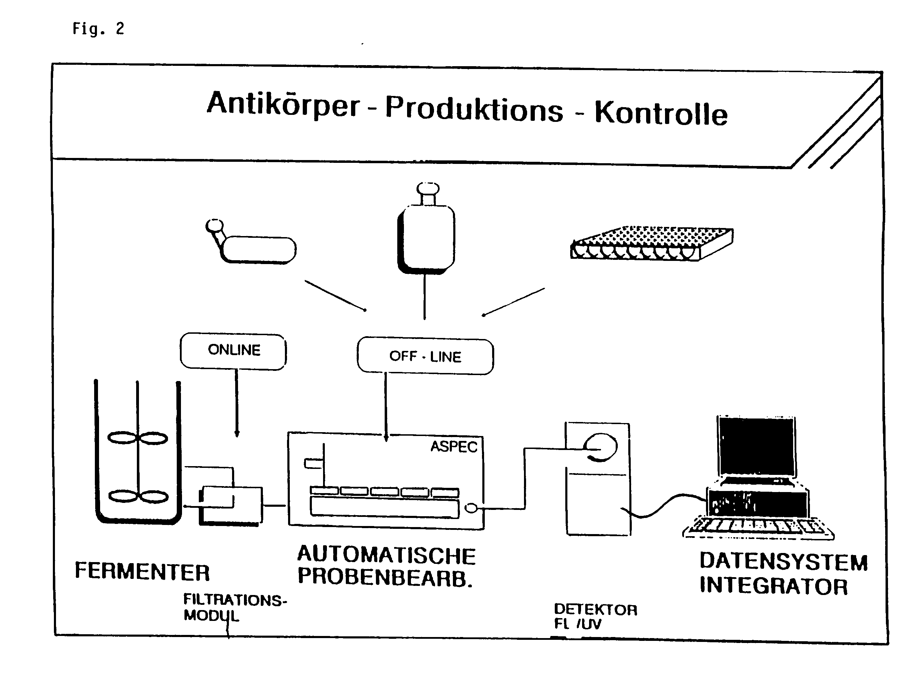

Die Erfindung wird nachfolgend mit Bezug auf die Fig. 1 und 2 näher erläutert. Es zeigen:
- Fig. 1a: einen Längsschnitt durch ein beispielhaftes Einwegreaktionsgefäß 1 mit dem Trägermaterial mit der immunologischen Reaktivkomponente 3, bei Bedarf mit zwei Trenneinrichtungen 2a und 2b,
- Fig. 1b: eine Draufsicht auf das Einwegreaktionsgefäß 1 und
- Fig. 2: eine schematische Darstellung eines On-Line-Systems zur Antikörper-Produktionskontrolle.

Die in den Figuren 1a und 1b wiedergegebenen Abmessungen sind lediglich beispielhaft.

Das Einwegreaktionsgefäß 1 sowie das Verfahren, bei dem das Einwegreaktionsgefäß 1 verwendet wird, bringen im Vergleich zu bekannten Reaktionsgefäßen bzw. Verfahren erhebliche Vorteile mit sich.

Das Einwegreaktionsgefäß 1 erlaubt eine rasche Durchführung der quantitativen Bestimmung von Komponenten, die über Immunreaktionen bestimmbar sind. Die Handhabung des Reaktionsgefäßes 1 ist denkbar einfach und erfordert nicht den Einsatz aufwendiger Apparaturen wie z.B. von HPLC-Apparaturen. Weiterhin werden ausgezeichnete Ergebnisse mit dem genannten Einwegreaktionsgefäß 1 erhalten bei einem äußerst geringen Trägermaterial-Volumen 3 und entsprechend geringem Verbrauch an der darauf aufgebrachten immunologischen Reaktivkomponente ( im folgenden als Trägerbett bezeichnet). Ein Einwegreaktionsgefäß 1 mit einem Trägerbettvolumen 8 von vorzugsweise 50 µl wird in dem erfindungsgemäßen Verfahren bevorzugt verwendet. Das geringe Trägerbettvolumen ermöglicht die Verwendung geringer Mengen an Elutionsflüssigkeit, was wiederum zur Folge hat, daß der Verdünnungseffekt durch die Elution gering bleibt, und somit die Nachweisgrenze im Vergleich zu anderen Verfahren, bei denen größere Elutionsvolumina notwendig sind, nicht verschlechtert wird. Durch Erhöhen des Volumenverhältnisses Probenlösung/Elutionspuffer, d.h. durch Aufgeben größerer Mengen von Probenlösung, kann die Nachweisgrenze noch weiter gesenkt werden.

Weiterhin ermöglicht das Reaktionsgefäß 1 die quantitative Bestimmung im Bindungsgleichgewicht (Endpunktbestimmung) zwischen zu bestimmender Komponente und der immunologischen Reaktivkomponente, wobei sich das Bindungsgleichgewicht bei Verwendung des Einwegreaktionsgefäßes 1 bereits innerhalb einer Inkubationszeit von einer Minute einstellt.
Das rasche Einstellen des Bindungsgleichgewichts wird durch das geringe Trägerbettvolumen 8, dessen Durchflußwiderstand sowie die hohe Beladung des Trägermaterials mit der immunologischen Reaktivkomponente ermöglicht. Der kontrollierte Durchfluß und die hohe Beladungsdichte des Trägermaterials mit der Reaktivkomponente haben zur Folge, daß die zu bestimmende Komponente innerhalb von ca. 1 min quantitativ (Bindungsgleichgewicht) an die Reaktivkomponente bindet, bevor die durchfließende Flüssigkeit aus dem Trägerbettvolumen austritt. Dadurch erübrigt sich eine Vergleichsanalytik mit Standardlösungen.

Eine schnelle Analyse von Proben wird weiterhin deshalb ermöglicht, weil das Einwegreaktionsgefäß 1 den sofortigen Gebrauch erlaubt, da das darin enthaltene Trägermaterial 3 mit der immunologischen Reaktivkomponente standardisiert und konfektioniert ist und das Einwegreaktionsgefäß 1 vor der Verwendung nicht geeicht werden muß.

Da das Einwegreaktionsgefäß 1 die rasche Analyse von Proben erlaubt, ohne den Einsatz der HPLC, muß das Trägermaterial 3 nicht druckstabil sein. Als Trägermaterial 3 kommen alle für die Affinitätschromatographie verwendbaren Materialien in Betracht. Bevorzugte Trägermaterialien sind polymere Zucker, Kunststoffe, kunststoff-modifizierte Träger, Metalloxide oder Silikate. Besonders bevorzugt sind Trägermaterialien in Form von Fritten. Für Nachweise von sehr großen Komponenten wie Viren, die keinen Zugang zu den Poren solcher Trägermaterialien haben, sind bevorzugt Propfcopolymere mit Seitenketten mit aktiven Gruppen. Die auf das Trägermaterial 3 aufgebrachte immunologische Reaktivkomponente kann sowohl kovalent als auch adsorptiv an das Trägermaterial gebunden sein. Die immunologische Reaktivkomponente kann ausgewählt werden aus der Gruppe umfassend Haptene, Antigene, Antikörper und immunologisch affine Proteine. Als Antikörper können sowohl polyklonale als auch monoklonale Antikörper verwendet werden.

Da das Einwegreaktionsgefäß 1 in dem erfindungsgemäßen Verfahren nicht hohen Drücken ausgesetzt werden muß, braucht das Gefäßmaterial 4 nicht druckstabil zu sein. Es kommen somit außer den druckstabilen Materialien, wie Metalle, andere nicht druckstabile Materialien, wie Glas, Naturstoffe und Kunststoffe, für die Herstellung des Einwegreaktionsgefäßes in Betracht. Bevorzugte Kunststoffe sind Polyethylen, Polypropylen und/oder Polystyrol, und bevorzugte Metalle sind Aluminium und Edelstahl.

Zweckmäßigerweise ist das Einwegreaktionsgefäß 1 an einem Ende mit einem Wulst 5 versehen, der das Einpassen des Reaktionsgefäßes in Halte- und Transportvorrichtungen zur automatischen Weiterbewegung ermöglicht. Dies ist erforderlich für den Einsatz in automatischen Probenbearbeitungsvorrichtungen. Weiterhin ist es zweckmäßig, daß das Einwegreaktionsgefäß 1 an dem einen Wulst tragenden gegenüberliegenden Ende einen Anschluß mit einem kleineren Durchmesser als dem Gefäßdurchmesser besitzt, vorzugsweise einen Anschluß, der eine Steckverbindung mit einem weiteren Einwegreaktionsgefäß ermöglicht. Dieser erleichtert u.a. das in Reihe-Schalten mehrerer Einwegreaktionsgefäße 1, wobei der Auslauf 6 des ersten Gefäßes mit dem Einlauf des zweiten Gefäßes durch einfaches Zusammenstecken der beiden Gefäße verbunden wird in Art einer Male-Female-Steckverbindung.

Weiterhin ist das Einwegreaktionsgefäß 1 durch Abdeckungen 7 und 9 abschließbar.

Das erfindungsgemäße Verfahren unter Verwendung des obigen Einwegreaktionsgefäßes 1 zeichnet sich u.a. dadurch aus, daß die Durchführung im Vergleich zu bisherigen Verfahren stark vereinfacht ist und es eine rasche quantitative und qualitative Bestimmung erlaubt. Dieses wird zum Teil deshalb ermöglicht, weil die Bestimmung der zu bestimmenden Komponente keine vorherige Kalibrierung oder Regenerierung des in dem Einwegreaktionsgefäß 1 verwendeten Trägermaterials erfordert und keine Vergleichsreihen mit Standardlösungen durchgeführt werden müssen. Die einfache Handhabung ergibt sich aus der Möglichkeit, Proben und weitere Lösungen mit üblichen Laborhilfsmitteln oder einem Pipettierautomaten aufzubringen.

Neben manuellen Verfahren werden auch automatisierte Verfahren durch den Einsatz des Reaktionsgefäßes 1 verbessert. Bei solchen Verfahren erfolgen das Aufbringen der Probe und die weiteren Verfahrensschritte durch eine automatische Probenbearbeitungsvorrichtung. Eine solche Probenbearbeitungsvorrichtung findet beispielsweise bei der Produktionskontrolle von in einem Fermenter hergestellen Antikörpern durch ein Online-System zur Prozeßkontrolle statt. Bei einem solchen Verfahren erfolgt die automatische Probenaufbringung in zeitlichen Abständen von etwa 6 bis 8 Stunden, und die in dem Fermentationsmedium enthaltene Antikörperkonzentration kann so kontinuierlich über einen längeren Zeitraum mit Hilfe des Einwegreaktionsgefäßes 1 rasch und einfach bestimmt werden. Eine schematische Darstellung eines Systems zur Antikörperproduktionskontrolle, bei dem das erfindungsgemäße Einwegreaktionsgefäß 1 eingesetzt werden kann, ist in Fig. 2 gezeigt. Dabei befindet sich das Einwegreaktionsgefäß 1 in der automatischen Probenbearbeitungsvorrichtung.

Das erfindungsgemäße Verfahren kann rasch durchgeführt werden, ohne daß das Einwegreaktionsgefäß 1 nach dem Aufbringen der Probe hohen Drücken ausgesetzt werden muß. Jedoch können zur schnelleren Durchführung des Verfahrens leichte Sog- oder Druckkräfte, wie sie z.B. durch Zentrifugation oder Pumpvorrichtungen zu erzeugen sind, angewendet werden.

Falls erforderlich, können bei dem erfindungsgemäßen Verfahren auch mehrere Einwegreaktionsgefäße 1 hintereinander oder parallel zueinander geschaltet werden. Dies erlaubt z.B. die gleichzeitige Bestimmung mehrerer Parameter in einer einzigen Probe bzw. die gleichzeitige Analyse mehrerer Proben.

Das Hintereinanderschalten mehrerer Einwegreaktionsgefäße ist z.B. bei der Durchführung von Allergietests besonders vorteilhaft. Dabei wird auf verschiedene Säulen jeweils ein unterschiedliches Antigen (Allergen) aufgebracht, die Säulen werden dann in Reihe geschaltet, und die Probe, enthaltend den die allergische Reaktion vermittelnden Antikörper der Klasse IgE, wird auf das erste Reaktionsgefäß aufgetragen. Die Probe durchläuft sukzessiv die verschiedenen, mit unterschiedlichen Allergenen bestückten Einwegreaktionsgefäße 1.

In dem Reaktionsgefäß, in dem das die Allergie hervorrufende Antigen vorhanden ist, wird das in der Probe enthaltene IgE gebunden und kann dort z.B. über Fluoreszenzdetektion nachgewiesen werden. Somit kann eine einzige Probe gleichzeitig mit einer Vielzahl verschiedener, potentieller Allergene ausgetestet werden.

## Patentansprüche

1. Oben und unten offenes Einwegreaktionsgefäß (1) für die Festphasenimmunanalytik mit mindestens einer auf ein Trägermaterial mit aktiver bzw. aktivierter Oberfläche (3) aufgebrachten immunologischen Reaktivkomponente mit einer derartigen Beladungsdichte, daß die zu bestimmende Komponente quantitativ (Bindungsgleichgewicht) an die Reaktivkomponente bindet, bevor die durchfließende Flüssigkeit aus dem Trägerbett austritt, wobei das Trägerbettvolumen (8) bis zu 600 µl beträgt, und wobei die Durchflußgeschwindigkeit der Flüssigkeit vom Durchflußwiderstand ausschließlich des Trägerbettes entsprechend bestimmt wird.

2. Einwegreaktionsgefäß nach Anspruch 1, dadurch gekennzeichnet, daß das Trägermaterial (3) mit der immunologischen Reaktivkomponente standardisiert und konfektioniert ist, was den sofortigen Gebrauch des Einwegreaktionsgefäßes (1) erlaubt.

3. Einwegreaktionsgefäß nach einem der Ansprüche 1 oder 2, dadurch gekennzeichnet, daß das Trägermaterial (3) ausgewählt wird aus der Gruppe umfassend polymere Zucker, Kunststoffe, kunststoffmodifizierte organische oder anorganische Träger, poröse Metalle und Legierungen, Metalloxide, Gläser, Silikate oder keramische Werkstoffe.

4. Einwegreaktionsgefäß nach Anspruch 3, dadurch gekennzeichnet, daß das Trägermaterial fest in Form einer Fritte oder Membran vorliegt, wobei die Porosität des Trägermaterials die Durchflußgeschwindigkeit der Flüssigkeit entsprechend Anspruch 1 bestimmt.

5. Einwegreaktionsgefäß nach Anspruch 3 und 4, dadurch gekennzeichnet, daß der Kunststoff eine Modifikation von Polyethylen, Polystyrol, Polyacrylat, Polyamid, von einem ihrer Copolymeren oder von Cellulose ist, daß das Metall Aluminium ist, und daß das Metalloxid Aluminiumoxid, Titandioxid oder Zirkondioxid ist,

6. Einwegreaktionsgefäß nach Anspruch 1 und 2, dadurch gekennzeichnet, daß das Trägermaterial eine signifikante Oberflächenstruktur mit hoher Konzentration an aktiven Bindungsstellen für die immunologische Reaktivkomponente aufweist, wobei die Durchflußgeschwindigkeit der Flüssigkeit von der Beziehung Teilchengröße und Oberflächenstruktur des Trägermaterials entsprechend Anspruch 1 bestimmt wird.

7. Einwegreaktionsgefäß nach Anspruch 6, dadurch gekennzeichnet, daß das Trägermaterial ein Propfcopolymer (Tentakelpolymer) ist, wobei die Durchflußgeschwindigkeit der Flüssigkeit vom Verhältnis zwischen Teilchengröße und Länge der Seitenketten entsprechend Anspruch 1 bestimmt wird.

8. Einwegreaktionsgefäß nach Anspruch 1, dadurch gekennzeichnet, daß die immunologische Reaktivkomponente kovalent oder adsorptiv an das Trägermaterial (3) gebunden ist.

9. Einwegreaktionsgefäß nach Anspruch 8, dadurch gekennzeichnet, daß die immunologische Reaktivkomponete ausgewählt wird aus der Gruppe umfassend Hapten, Antigen, Antikörper und immunologisch affine Proteine.

10. Einwegreaktionsgefäß nach Anspruch 9, dadurch gekennzeichnet, daß der Antikörper polyklonal ist.

11. Einwegreaktionsgefäß nach Anspruch 9, dadurch gekennzeichnet, daß der Antikörper monoklonal ist.

12. Einwegreaktionsgefäß nach Anspruch 4, dadurch gekennzeichnet, daß das Trägermaterial eine Porosität von 0,2 µm bis 100 µm aufweist.

13. Einwegreaktionsgefäß nach Anspruch 4, dadurch gekennzeichnet, daß das Trägermaterial eine Schichtdicke von 0,1 mm bis 20 mm aufweist.

14. Einwegreaktionsgefäß nach Anspruch 7, dadurch gekennzeichnet, daß das Propfcopolymer Polystyrol mit aufgepropftem Polyethylenglykol ist mit einer Teilchengröße von 10 bis 15 µm.

15. Einwegreaktionsgefäß nach einem der vorhergehenden Ansprüche 1 bis 14, dadurch gekennzeichnet, daß das Gefäßmaterial (4) ausgewählt wird aus der Gruppe umfassend Kunststoffe, Gläser, Metalle und Naturstoffe.

16. Einwegreaktionsgefäß nach Anspruch 15, dadurch gekennzeichnet, daß der Kunststoff Polyethylen, Polypropylen und/oder Polystyrol ist.

17. Einwegreaktionsgefäß nach einem der vorhergehenden Ansprüche 1 bis 16, dadurch gekennzeichnet, daß das Reaktionsgefäß (1) an einem Ende einen Wulst (5) trägt, der das Einpassen des Reaktionsgefäßes in Halte- und Transporteinrichtungen zur automatischen Weiterbewegung ermöglicht.

18. Einwegreaktionsgefäß nach einem der vorhergehenden Ansprüche 1 bis 17, dadurch gekennzeichnet, daß das Reaktionsgefäß (1) an dem einen Wulst (5) tragenden gegenüberliegenden Ende einen Anschluß (6) mit einem kleineren Durchmesser als dem Gefäßdurchmesser besitzt.

19. Einwegreaktionsgefäß nach Anspruch 18, dadurch gekennzeichnet, daß die gegenüberliegenden Enden des Reaktionsgefäßes ein Zusammenkoppeln in Art einer Male-Female-Steckverbindung ermöglichen zum Hintereinanderschalten mehrerer Einwegreaktionsgefäße.

20. Einwegreaktionsgefäß nach einem der vorhergehenden Ansprüche 1 - 19, dadurch gekennzeichnet, daß das Trägerbettvolumen (8) 50 µl beträgt.

21. Verfahren zur Bestimmung von über eine Immunreaktion bestimmbaren Komponenten, dadurch gekennzeichnet, daß auf ein Einwegreaktionsgefäß nach einem der Ansprüche 1 bis 20 eine zu analysierende Probe gegeben wird, die zu bestimmende Komponente in der Probe von der komplementären immunologischen Reaktivkomponente in dem Einwegreaktionsgefäß während des direkten Durchflusses zurückgehalten wird, und die zu bestimmende Komponente
a) eluiert und bestimmt wird, oder
b) im Einwegreaktionsgefäß über Verstärkerreaktionen mit immunologischen Markern markiert und dann bestimmt wird, oder
c) über eine zu der zu bestimmenden Komponente konkurrierende markierte Verbindung bestimmt wird.

22. Verfahren nach Anspruch 21, dadurch gekennzeichnet, daß die Bestimmung ohne vorherige Kalibrierung oder Regenerierung des Trägermaterials und der darauf aufgebrachten Reaktivkomponente durchgeführt wird.

23. Verfahren nach Anspruch 21 oder 22, dadurch gekennzeichnet, daß das Aufbringen der Probe und weiterer Lösungen mit üblichen Laborhilfsmitteln oder einem Pipettierautomaten erfolgt.

24. Verfahren nach einem der Ansprüche 21 oder 22, dadurch gekennzeichnet, daß das Aufbringen der Probe und die weiteren Verfahrensschritte durch eine automatische Probenbearbeitungsvorrichtung durchgeführt werden.

25. Verfahren nach Anspruch 24, dadurch gekennzeichnet, daß die automatische Probenaufbringung in einem On-line-Prozeßkontroll-System erfolgt.

26. Verfahren nach einem der Ansprüche 21 bis 25, dadurch gekennzeichnet, daß zur schnelleren Durchführung des Verfahrens das Einwegreaktionsgefäß nach dem Aufbringen der Probe leichten Sog- oder Druckkräften ausgesetzt wird.

27. Verfahren nach einem der Ansprüche 21 bis 26, dadurch gekennzeichnet, daß mehrere Einwegreaktionsgefäße hintereinander oder parallel geschaltet werden.

28. Verfahren nach Anspruch 27, dadurch gekennzeichnet, daß damit Allergene bestimmt werden.

## Claims

1. Disposable reaction vessel open at the top and bottom (1) for solid-phase immunoanalytics having at least one immunological reactive component provided on a support material with an active or activated surface (3) with such a loading density that the component to be assayed will quantitatively (binding equilibrium) bind to said reactive component before the liquid flowing through exits from the support bed, the volume (8) of the support bed being up to 600 µl, and the flow rate of the liquid being correspondingly determined exclusively by the flow resistance of the support bed.

2. The disposable reaction vessel according to claim 1, characterized in that said support material (3) is standardized and with the immunological reactive component and ready-made, allowing the disposable reaction vessel (1) to be immediately used.

3. The disposable reaction vessel according to either of claims 1 or 2, characterized in that said support material (3) is selected from the group comprising polymeric sugars, plastic materials, plastic-modified organic or inorganic supports, porous metals and alloys, metal oxides, glasses, silicates or ceramic materials.

4. The disposable reaction vessel according to claim 3, characterized in that said support material is present in solid form as a frit or membrane, the porosity of the support material determining the flow rate of the liquid in accordance with claim 1.

5. The disposable reaction vessel according to claims 3 and 4, characterized in that said plastic material is a modification of polyethylene, polystyrene, polyacrylate, polyamide, of one of their copolymers, or of cellulose, said metal is aluminium, and said metal oxide is aluminium oxide, titanium dioxide or zirconium dioxide.

6. The disposable reaction vessel according to claims 1 and 2, characterized in that said support material has a significant surface structure with a high concentration of active binding sites for said immunological reactive component, the flow rate of the liquid being determined by the relationship between particle size and surface structure of the support material in accordance with claim 1.

7. The disposable reaction vessel according to claim 6, characterized in that said support material is a graft copolymer (tentacle polymer), the flow rate of the liquid being determined by the ratio of particle size to length of the side-chains in accordance with claim 1.

8. The disposable reaction vessel according to claim 1, characterized in that said immunological reactive component is covalently or adsorptively bound to said support material (3).

9. The disposable reaction vessel according to claim 8, characterized in that said immunological reactive component is selected from the group comprising a hapten, an antigen, an antibody and immunological affinity proteins.

10. The disposable reaction vessel according to claim 9, characterized in that said antibody is a polyclonal antibody.

11. The disposable reaction vessel according to claim 9, characterized in that said antibody is a monoclonal antibody.

12. The disposable reaction vessel according to claim 4, characterized in that said support material has a porosity of from 0.2 µm to 100 µm.

13. The disposable reaction vessel according to claim 4, characterized in that said support material has a layer thickness of from 0.1 mm to 20 mm.

14. The disposable reaction vessel according to claim 7, characterized in that said graft copolymer is polystyrene with grafted polyethylene glycol having a particle size of from 10 to 15 µm.

15. The disposable reaction vessel according to any of the preceding claims 1 to 14, characterized in that the vessel material (4) is selected from the group comprising plastics, glasses, metals and natural substances.

16. The disposable reaction vessel according to claim 15, characterized in that said plastic is polyethylene, polypropylene and/or polystyrene.

17. The disposable reaction vessel according to any of the preceding claims 1 to 16, characterized in that said reaction vessel (1) bears a bead (5) at one end thereof allowing the reaction vessel to be engaged in holding and transporting devices for automated conveying.

18. The disposable reaction vessel according to any of the preceding claims 1 to 17, characterized in that said reaction vessel (1) has a connection (6) with a diameter smaller than the vessel diameter at the end opposite that bearing a bead (5).

19. The disposable reaction vessel according to claim 18, characterized in that the opposing ends of the reaction vessel permit coupling of the kind of a male-female plugging connection for connecting several disposable reaction vessels in series.

20. The disposable reaction vessel according to any of the preceding claims 1 to 19, characterized in that said volume (8) of the support bed is 50 µl.

21. A method for assaying components which can be determined by an immune reaction, characterized in that a sample to be analyzed is charged on a disposable reaction vessel according to any of claims 1 to 20, the component to be assayed in the sample is retained by the complementary immunologic reactive component in the disposable reaction vessel while the sample directly flows through, and the component to be assayed is
a) eluted and determined; or
b) labeled within the disposable reaction vessel via enhancement reactions with immunological markers and then determined; or
c) determined via a compound competing with the component to be assayed.

22. The method according to claim 21, characterized in that said determination is performed without prior calibration or regeneration of the support material and the reactive component provided thereon.

23. The method according to claim 21 or 22, characterized in that the application of the sample and of further solutions is effected with usual laboratory accessories or with an automatic pipetting device.

24. The method according to either of claims 21 or 22, characterized in that the application of the sample and the further process steps are performed by an automatic sample processing device.

25. The method according to claim 24, characterized in that said the automatic sample application is performed in an on-line process control system.

26. The method according to any of claims 21 to 25, characterized in that said disposable reaction vessel is subjected to gentle sucking or pressing forces after the application of the sample for the method to be performed more quickly.

27. The method according to any of claims 21 to 26, characterized in that several disposable reaction vessels are connected in series or in parallel.

28. The method according to claim 27, characterized in that allergens are assayed therewith.

## Revendications

1. Tube de réaction jetable (1) ouvert à son niveau supérieur et à son niveau inférieur, utilisé pour l'analyse immunologique en phase solide, comportant au moins un composant réactif immunologique appliqué sur une matière de support (3), comportant une surface active ou bien activée, avec une densité de charge telle que le composant à déterminer se lie de façon quantitative (équilibre de liaison) au composant réactif, ceci avant le passage du liquide au travers du lit de support, ledit lit de support (8) ayant jusqu'à 600 µl de volume et la vitesse de passage du liquide étant uniquement déterminée par la résistance de passage liée au lit de support.

2. Tube de réaction jetable selon la revendication 1 caractérisé en ce que la matière de support (3) est standardisée et confectionnée avec le composant réactif immunologique, ce qui permet l'utilisation directe dudit tube de réaction jetable (1).

3. Tube de réaction jetable, selon l'une quelconque des revendications 1 ou 2, caractérisé en ce que la matière de support (3) est sélectionnée parmi le groupe comprenant des polymères, sucres, matières plastiques, supports organiques ou inorganiques modifiés pae des matières plastiques, métaux et alliages poreux, oxydes métalliques, verres, silicates ou bien matériaux céramiques.

4. Tube de réaction jetable selon la revendication 3, caractérisé en ce que la matière de support est solide sous forme de membrane ou fritte, la porosité de la matière de support déterminant la vitesse de passage du liquide selon la revendication 1.

5. Tube de réaction jetable selon l'une quelconque des revendications 3 et 4, caractérisé en ce que la matière plastique est une modification du polyéthylène, polystyrène, polyacrylate, polyamide, de l'un de leurs copolymères ou bien de la cellulose, et en ce que le métal est de l'aluminium, et en ce que l'oxyde métallique est de l'oxyde d'aluminium, de l'oxyde de titane, ou de l'oxyde de zirconium.

6. Tube de réaction jetable selon l'une quelconque des revendications 1 et 2, caractérisé en ce que la matière de support présente une structure de surface caractéristique ayant une concentration élevée de sites actifs de liaison pour le composant réactif, la vitesse du passage du liquide étant déterminée par le rapport entre la taille des particules et la structure de la surface de la matière de support selon la revendication 1.

7. Tube de réaction jetable, selon la revendication 6, caractérisé en ce que la matière de support est un copolymère greffé (polymère tentacule), la vitesse du passage du liquide étant déterminée par le rapport entre la taille des particules et la longueur des chaînes ramifiées selon la revendication 1.

8. Tube de réaction jetable selon la revendication 1, caractérisé en ce que le composant réactif immunologique est lié d'une façon covalente ou bien par adsorption à la matière de support (3).

9. Tube de réaction jetable selon la revendication 8, caractérisé en ce que le composant réactif immunologique est selectionné parmi les éléments du groupe comprenant un haptène, un antigène, un anticorps et des protéines affinées d'une façon immunologique.

10. Tube de réaction jetable selon la revendication 9, caractérisé en ce que l'anticorps est polyclonal.

11. Tube de réaction jetable selon la revendication 9, caractérisé en ce que l'anticorps est monoclonal.

12. Tube de réaction jetable selon la revendication 4, caractérisé en ce que la la matière de support a une porosité comprise entre 0,2 µm et 100 µm.

13. Tube de réaction jetable selon la revendication 4, caractérisé en ce que la matière de support a une épaisseur de couche comprise entre 0,1 mm et 200 mm.

14. Tube de réaction jetable selon la revendication 7, caractérisé en ce que le copolymère greffé est un polystyrène greffé d'un polyéthylène glycol, ayant une taille de particule comprise entre 10 µm et 15 µm.

15. Tube de réaction jetable selon l'une quelconque des revendications 1 à 14, caractérisé en ce que le matériau du tube (4) est choisi parmi les éléments du groupe comprenant des matières plastiques, des verres, des métaux et des matières naturelles.

16. Tube de réaction jetable selon la revendication 15, caractérisé en ce que la matière plastique est du polyéthylène ou polypropylène et/ou polystyrène.

17. Tube de réaction jetable selon l'une quelconque des revendications 1 à 16, caractérisé en ce que le tube de réaction (1) comporte à l'une de ses extrémités un anneau (5), permettant d'emboîter le tube de réaction (1) dans des moyens de support et de transport en vue d'un déplacement ultérieur automatique.

18. Tube de réaction jetable selon l'une quelconque des revendications 1 à 17, caractérisé en ce que le tube de réaction (1) comporte une sortie (6) ayant un diamètre inférieur au diamètre du tube, disposé à l'extrémité opposée à l'anneau (5).

19. Tube de réaction jetable selon la revendication 18, caractérisé en ce que les deux extrémités opposées du tube de réaction permettent leur couplage par une communication mâle-femelle, afin de pouvoir monter en série plusieurs tubes de réaction jetables.

20. Tube de réaction jetable selon l'une quelconque des revendications 1 à 19, caractérisé en ce que le lit de support (8) a un volume de 50 µl.

21. Procédé pour la détermination d'un composant susceptible d'être déterminé par une réaction immunologique, caractérisé en ce que l'on met un échantillon soumis à détermination dans le tube de réaction jetable selon l'une quelconque des revendications 1 à 20, le composant à déterminer chargé dans l'échantillon est retenu par le composant réactif immunologique complémentaire à l'intérieur du tube de réaction lors du passage direct, et le composant à déterminer est soumis à:
(a) une élution et une détermination, ou bien
(b) un marquage à l'aide de marqueurs immunologiques par la voie de réactions amplifiées dans le tube de réaction, et ensuite à une détermination, ou bien
(c) la détermination d'un composant marqué, en compétition avec ledit composant à déterminer.

22. Procédé selon la revendication 21, caractérisé en ce que la détermination est effectuée sans avoir préalablement à calibrer ou bien régénérer la matière de support ou le composant réactif appliqué dessus.

23. Procédé selon l'une quelconque des revendications 21 ou 22, caractérisé en ce que l'application de l'échantillon et d'autres solutions est effectuée à l'aide des moyens de laboratoire habituels ou bien d'un appareillage à pipette.

24. Procédé selon l'une quelconque des revendication 21 ou 22, caractérisé en ce que l'application de l'échantillon et les étapes suivantes du procédé sont effectuées par un appareillage automatique de manipulation des échantillons.

25. Procédé selon la revendication 24, caractérisé en ce que la mise en place automatique de l'échantillon est effectuée par un système en ligne de contrôle d'opérations.

26. Procédé selon l'une quelconque des revendications 21 à 25, caractérisé en ce que le tube de réaction jetable est soumis à de faibles forces de dépression ou de pression après mise en place de l'échantillon, afin d'accélérer la réalisation du procédé.

27. Procédé selon l'une quelconque des revendications 21 à 26, caractérisé en ce que plusieurs tubes de réaction jetables sont disposés en série ou en parallèle.

28. Procédé selon 27, caractérisé en ce que celui-ci sert à déterminer des allergènes.
